# FASCICULE DE BREVET EUROPEEN

(11) **EP 1 965 864 B1**
(45) Date de publication et mention de la délivrance du brevet: **21.09.2011**
(21) Numéro de dépôt: 06847168.9
(22) Date de dépôt: 18.12.2006
(51) Int. Cl.: A61P 17/00, A61P 17/10, A61K 9/107, A61K 31/35

(54) **COMPOSITION DE TYPE EMULSION INVERSE COMPRENANT DE L'IVERMECTINE, ET SES UTILISATIONS EN COSMETIQUE ET EN DERMATOLOGIE**
INVERSE EMULSIONSZUSAMMENSETZUNG MIT IVERMECTIN UND VERWENDUNG DAVON IN DER KOSMETIK UND IN DER DERMATOLOGIE
COMPOSITION OF INVERSE EMULSION TYPE COMPRISING IVERMECTIN, AND USES THEREOF IN COSMETICS AND DERMATOLOGY

(30) Priorité: 20.12.2005 FR 0512956
(43) Date de publication de la demande: 10.09.2008
(73) Titulaire: Galderma S.A., 6330 Cham (CH)
(72) Inventeur: SEGURA-ORSONI, Sandrine, F-06210 Mandelieu (FR); DIAZ-ASTRUC, Fanny, Houston, TX 77077 (US)
(74) Mandataire: Allab, Myriam
(86) Numéro de dépôt international: PCT/FR2006/051378
(87) Numéro de publication internationale: WO 2007/071876

(56) Documents cités:
- EP-A- 0 045 655
- WO-A-2004/093886
- WO-A-2005/089806
- US-A1- 2003 180 350

## Description

La présente invention se rapporte à une nouvelle composition de type émulsion inverse comprenant au moins un composé de la famille des avermectines, de préférence l'ivermectine, pour la fabrication d'une composition pharmaceutique topique destinée au traitement de la rosacée.

L'ivermectine est un mélange de deux composés appartenant à la classe des avermectines, la 5-0-demethyl-22,23-dihydroavermectine A₁ₐ et la 5-0-demethyl-22,23-dihydroavermectine A_{1b}. Ils sont également connus sous le nom de 22,23-dihydroavermectine B₁ₐ et 22,23-dihydroavermectine B_{1b}. L'ivermectine contient au moins 80% de 22,23-dihydroavermectine B₁ₐ et moins de 20% de 22,23-dihydroavermectine B_{1b}. Cet agent actif fait partie de la classe des avermectines, un groupe de lactones macrocycliques produit par le bacterium *Streptomyces avermitilis* (Reynolds JEF (Ed) (1993) Martindale. The extra pharmacopoeia. 29th Edition. Pharmaceutical Press, London).

Au milieu des années 80, l'ivermectine a été présenté comme médicament anti-parasitaire de large spectre à usage vétérinaire (CAMPBELL, W.C., et al., (1983). Ivermectin: a potent new antiparasitic agent. Science, 221, 823-828.). Il est efficace contre la plupart des vers intestinaux communs (excepté les ténias), la plupart des acarides, et quelques poux. Il présente notamment, une affinité importante pour les canaux chlore glutamate-dépendants présents dans les cellules nerveuses et musculaires des invertébrés. Sa fixation sur ces canaux favorise une augmentation de la perméabilité membranaire aux ions chlores, entraînant une hyperpolarisation de la cellule nerveuse ou musculaire. Il en résulte une paralysie neuromusculaire pouvant entraîner la mort de certains parasites. L'ivermectine interagit également avec d'autres canaux chlorure ligand-dépendants tels que ceux faisant intervenir le neuromédiateur GABA (acide gamma-aminobutyrique).

L'ivermectine est plus particulièrement un antihelminthique. Il est déjà décrit chez l'homme dans le traitement de l'onchocercose à Onchocerca volvulus, de la strongyloïdose (anguillulose) gastro-intestinale (produit Stromectol®), de la gale sarcoptique humaine (Meinking TL et al., N Engl J Med 1995 Jul 6;333(1):26-30 The treatment of scabies with ivermectin) ainsi que dans le traitement de la microfilarémie diagnostiquée ou suspectée chez les sujets atteints de la filariose lymphatique due à Wuchereria bancrofti.

La demande de brevet EP045655 divulgue que l'ivermectine, qui est instable et insoluble dans l'eau, peut être solubilisée par la formation de particules colloïdales appelées micelles.

La demande de brevet US2003/0180350 divulgue des compositions vétérinaires pouvant comprendre dans la même formulation un actif hydrophile et un actif lipophile, qui peut être notamment une avermectine, et des mono ou di-glycérides à chaîne moyenne.

Le brevet US 6,133,310 divulgue l'utilisation de l'ivermectine en voie topique sous forme de prototype d'une lotion constituée d'un mélange d'ivermectine et d'eau et évoque également la possibilité d'un prototype d'une crème constituée quant à elle du mélange de l'ivermectine et d'un excipient tel que le propylène glycol ou le sodium lauryl sulfate mais ne décrit aucune composition pharmaceutique en tant que telle. Ces mélanges s'apparentent à des préparations expérimentales utilisées dans le cadre de premiers résultats d'une preuve de concept. En effet, les éléments divulgués dans ce brevet ne donnent aucun enseignement à l'homme du métier sur la faisabilité de compositions pharmaceutiques contenant de l'ivermectine industriellement acceptables, notamment ayant une bonne cosméticité et une péremption suffisante pour un produit pharmaceutique industriel (minimum 2 ans).

L'ivermectine selon l'invention contient au moins 80% de 22,23-dihydroavermectine B₁ₐ et moins de 20% de 22,23-dihydroavermectine B_{1b}.

L'ivermectine présente une grande instabilité à la présence d'eau et il s'avère particulièrement difficile d'obtenir des compositions pharmaceutiques stables contenant l'ivermectine. Elle présente la difficulté d'être très faiblement soluble et rarement stable dans les solvants cosmétiques ou pharmaceutiques couramment utilisés, notamment l'eau ; elle est en effet sensible à un environnement aqueux. Cette sensibilité à l'eau peut conduire à une instabilité chimique de l'actif et/ou à une cristallisation de l'actif initialement solubilisé. Cette sensibilité à l'eau limite donc sa formulation dans des compositions cosmétiques ou dermatologiques appliquées par voie topique ou orale. Les phénomènes de dégradation chimique et/ou de cristallisation de l'ivermectine en présence d'eau ont pour conséquence une diminution ou une perte d'efficacité et une incertitude quant à la dose d'actif mise en oeuvre lors de son utilisation, ce qui va à l'encontre de l'objectif recherché. En outre, cette dégradation de l'actif et/ou sa cristallisation peuvent modifier la stabilité globale des compositions ainsi que leur aspect.

La forme galénique la plus couramment utilisée aujourd'hui en dermatologie est l'émulsion huile dans eau dans laquelle l'actif est préférentiellement solubilisé dans la phase lipophile. Mais cette solution reste peu satisfaisante car pour répondre à un objectif de concentration en actif ayant une efficacité thérapeutique quantifiable, il faudrait des concentrations en huiles solvantes très élevées, conduisant à des produits sans aucun doute peu agréables à utiliser, dû à leur toucher collant, instables physiquement et tout en restant limités en concentration d'actif.

Une possibilité est de solubiliser l'actif dans la phase hydrophile de l'émulsion, dans la limite de sa solubilité dans les milieux aqueux ou hydroglycoliques.

Mais cette solution ne permet pas de résoudre les problèmes de stabilité chimique rencontrés avec l'ivermectine, car l'activité en eau de l'émulsion reste très élevée.

La substitution de tout ou partie de la phase aqueuse par un ou plusieurs glycols conduit généralement à des formulations cosmétiquement peu acceptables. En particulier, au delà de 20% de glycol, la formulation est cosmétiquement peu acceptable de par son toucher collant, et sa stabilité physique n'est souvent pas garantie.

Cependant, de façon surprenante, la demanderesse a réussit à substituer une partie importante de la phase aqueuse par des glycols sans pour autant rencontrer les inconvénients cités ci-dessus.

La demanderesse a déjà protégé dans sa demande FR2867684, ou la demande W02005/089806, des compositions pharmaceutiques sous forme de gel-crème comprenant de l'Ivermectine et un émulsionnant polymérique non-surfactant tel que les Pemulen ou les carbopols. La demanderesse a décrit également dans la demande WO 2004/093886 des compositions sous forme d'émulsions huile dans eau, de gels ou de solutions, comprenant de l'ivermectine, une phase huileuse, une phase aqueuse et une phase active solvante de l'actif pouvant contenir des glycols.. Mais il reste important d'améliorer encore la stabilité des compositions comprenant l'ivermectine tout en préservant la tolérance de la composition.

La Demanderesse a constaté de façon surprenante que la composition de type émulsion inverse selon l'invention présente une très bonne stabilité physique et chimique, et une très bonne tolérance sur la peau. En effet, il s'avère qu'elle est particulièrement adaptée au traitement des affections dermatologiques et plus particulièrement bien adaptée pour le traitement de la rosacée.

La présente invention a également pour objet un procédé d'obtention de la composition selon l'invention et son utilisation pour la fabrication d'un médicament topique destiné au traitement de la rosacée.

L'élaboration d'une émulsion inverse comme alternative n'était pas évidente pour l'homme du métier compte tenu des difficultés connues de formuler l'ivermectine dans des compositions stables. Par émulsion inverse, on entend une émulsion de type phase hydrophile dispersée dans phase lipophile.

L'utilisation d'agents solubilisants hydrophiles comme le propylène glycol n'était également pas naturelle pour l'homme du métier compte tenu du fait que les fortes concentrations nécessaires n'étaient pas favorables à une bonne stabilité physique de la formule et à un toucher cosmétique acceptable.

L'obtention d'une bonne tolérance avec des solubilisants comme le propylène glycol n'était également pas évidente car il a été montré chez l'homme des phénomènes d'intolérance cutanée, par exemple chez l'homme sain (Motoyoshi et al, Cosmet and toiletries, 99, 83-89, 1984).

Il existait donc un besoin d'une composition permettant de répondre à un ou plusieurs des aspects suivants : disposer d'une bonne stabilité de la formule au froid et à la chaleur, en particulier quant au maintien de la taille des globules et à l'absence de déphasage ainsi qu'une viscosité stable dans le temps, avoir une bonne résistance de l'ivermectine vis-à-vis des phénomènes d'oxydation, permettre une bonne stabilité chimique de l'actif et une bonne disponibilité de celui-ci pour la peau, présenter une bonne tolérance cutanée. Il est également utile de pouvoir disposer d'une composition autorisant une forte fraction volumique dispersée. Il est par ailleurs utile que la préparation de telles compositions bénéficie d'un mode de préparation avantageux.

Or la Demanderesse a mis au point de façon surprenante une formulation de type glycol dans huile qui permet de s'affranchir des différents problèmes liés aux aspects mentionnés ci-dessus en permettant notamment de disposer d'une bonne stabilité physique de la composition en tant que telle, mais aussi de permettre une bonne stabilité chimique et disponibilité de l'actif, notamment l'ivermectine, qu'elle contient. La composition selon l'invention a également l'avantage de présenter une bonne tolérance cutanée et d'autoriser une forte fraction volumique dispersée.

L'invention se rapporte donc à une composition comprenant au moins un composé de la famille des avermectines, de préférence l'ivermectine, caractérisée en ce que la composition est une émulsion inverse contenant une phase hydrophile dispersée glycolique ou hydroglycolique, une phase continue lipophile et un émulsionnant de HLB compris entre 2 et 7, ladite composition ne comprenant pas de DHEA et/ou ses précurseurs et/ou dérivés chimiques et/ou biologiques et/ou de dérivé de vitamine D.

Les composés de la famille des avermectines utilisables selon la présente invention incluent notamment l'invermectine, l'ivermectine, l'avermectine, l'abamectine, la doramectine, l'eprinomectine et la sélamectine. De manière préférentielle, le composé de la famille des avermectines est l'ivermectine.

La composition selon l'invention comprend de préférence exclusivement le composé de la famille des avermectines, de préférence l'ivermectine, à titre de principe actif.

Par le terme HLB, on entend le Rapport Hydrophile/Lipophile ou « Hydrophilic/Lipophilid Balance (HLB) » qui correspond à l'équilibre entre la dimension et la force du groupe hydrophile et la dimension et la force du groupe lipophile de l'émulsionnant.

La composition selon l'invention permet d'obtenir de bonnes propriétés de libération/pénétration de l'actif, notamment l'ivermectine, au niveau des différentes assises cutanées, conduisant à une bonne disponibilité de l'actif dans la peau, celui-ci étant présent sous forme solubilisée.

Par forme solubilisée, on entend une dispersion de l'actif à l'état moléculaire dans un liquide, aucune cristallisation de l'actif n'étant visible à l'oeil nu ni même au microscope optique en polarisation croisée.

La formulation de l'ivermectine solubilisée dans une phase glycolique ou hydroglycolique en émulsion inverse selon l'invention permet ainsi, de manière surprenante, de s'affranchir des problèmes de stabilité chimique et de cristallisation couramment rencontrés en formulation avec le type d'actif utilisé selon l'invention.

La présente invention se rapporte également à la préparation d'émulsions inverses, contenant une phase hydrophile glycolique ou hydroglycolique, parfaitement stables chimiquement et physiquement, même à forte fraction volumique dispersée, ne montrant aucune dégradation chimique et/ou cristallisation de l'ivermectine et particulièrement bien tolérées.

Par stabilité physique selon l'invention, on entend une composition ne présentant aucune modification d'aspect macroscopique (séparation de phase, changement de couleur d'aspect, viscosité, etc..) ni microscopique (recristallisation des actifs) après stockage aux températures de 4°C, 25°C, 40°C et 55°C pendant 3 mois.

Par stabilité chimique selon l'invention, on entend une composition dans laquelle la teneur en principe actif reste stable après trois mois à température ambiante et à 40 °C. Une teneur stable en principe actif signifie selon l'invention que la teneur présente très peu de variation par rapport à la teneur initiale, c'est-à-dire que la variation de teneur en principe actif au temps T ne doit pas être inférieure à 90% et plus particulièrement à 95% de la teneur initiale à T0.

De préférence, le composé de la famille des avermectines, de préférence l'ivermectine, est solubilisé dans la phase dispersée glycolique ou hydroglycolique de l'émulsion, inverse selon l'invention. De manière plus avantageuse, le composé de la famille des avermectines, notamment l'ivermectine, est exclusivement solubilisé dans le(s) glycol(s) utilisé(s) dans l'émulsion inverse selon l'invention.

La composition selon l'invention est de préférence adaptée à une application topique sur la peau, les phanères et/ou les muqueuses. Elle comprend généralement un milieu physiologiquement acceptable et une quantité de composé de la famille des avermectines, notamment l'ivermectine, suffisante pour obtenir l'effet recherché. La proportion pondérale de composé de la famille des avermectines, de préférence l'ivermectine, par rapport au poids total de la composition peut ainsi être comprise entre 0,001% et 20% en poids par rapport au poids total de la composition, de préférence entre 0,01 et 5%, en particulier entre 0,02 et 2%. Plus particulièrement, la composition contient 1% en poids d'actif, notamment l'ivermectine, par rapport au poids total de la composition.

Les glycols à considérer dans la présente invention peuvent être définis comme des alkylène ou des poly alkylène glycols. A titre d'exemples non limitatifs, on peut citer les alkylènes et polyalkylènes glycols (C1 à C6) tel que l'éthylène glycol, le polyéthylène glycol (2 à 20 monomères), le propylène glycol, le dipropylène glycol, le butylène glycol, le pentylène glycol, l'hexylène glycol. Ils peuvent être oxyéthylénés ou non (2 à 50 OE). Les préférés selon l'invention, sont l'hexylène glycol, le propylène glycol et le dipropylène glycol, et le polyéthylène Glycol 400 (PEG 400).

Les glycols utilisables selon l'invention auront avantageusement comme paramètre de solubilité un δp inférieur à 10, étant entendu que les 3 paramètres de solubilité de Hansen : δd, δp et δh caractérisent, pour un constituant donné, les énergies correspondant respectivement aux interactions dispersives, polaires et de type liaisons hydrogène existant entre les molécules de ce constituant, δp caractérisant plus particulièrement les forces d'interaction de Debye entre dipôles et étant fonction du nombre d'atomes d'oxygène dans la formule du constituant donné (S. paint Technology, 30, 195, 1967, « The three dimensional solubility parameter -Key to paint component affinities »). De préférence, les paramètres de solubilité des glycol préférés selon l'invention sont compris entre 5 et 10.

De préférence, la phase hydrophile dispersée comprend au moins un glycol choisi parmi le propylène glycol, l'hexylène glycol, le dipropylène glycol et le PEG 400.

Comme composés lipophiles utilisables pour constituer la phase grasse continue des émulsions selon l'invention, on peut citer les huiles minérales (huile de paraffine), les huiles d'origine végétale (huile d'avocat, huile d'amande douce, huile de soja), les huiles d'origine animale (lanoline), les huiles de synthèse (perhydrosqualène), les huiles siliconées (cyclométhicone, diméthicone) et les huiles fluorées (perfluoropolyéthers). On peut également utiliser des alcools gras tels que l'alcool cétylique, les alcools de Guerbet, en particulier l'octyldodecanol connu sous l'appelation Eutanol G, des acides gras, des cires, des gommes et en particulier les gommes de silicone.
La phase grasse pourra également être constituée de mono, di ou triesters d'origine synthétique, linéaires ou ramifiés, en particulier myristate ou palmitate d'isopropyle, ou caprylic/capric triglycéride (Miglyol 812).

De façon préférée, on utilise des composés non oxydables pour constituer les huiles de la phase lipophile continue, qui sont préférentiellement choisies parmi celles de type siliconé, celles de type ester ou celles de type minéral.

Les composés entrant dans la composition de la phase lipophile de l'émulsion auront comme paramètre de solubilité de Hansen un δp inférieur à 5, et par exemple compris entre 0 et 2.

D'autre part, afin d'éviter toute cristallisation de l'ivermectine, le paramètre de solubilité global de la phase lipophile : δtₜ= √δd+ δp + δh aura une valeur inférieure à 18, par exemple entre 10 et 18, et de préférence entre 12 et 18

La fraction volumique de la phase hydrophile dispersée dans l'émulsion selon l'invention va de 10 à 90% par rapport au volume total de l'émulsion. Elle peut être exclusivement glycolique ou hydroglycolique.

La proportion volumique de glycols par rapport au volume total de la phase hydrophile dispersée se situe entre 30 et 100% et de préférence entre 60 et 100%.

Les émulsionnants (ou tensio-actifs ou surfactants) sont des substances naturelles ou synthétiques formées d'une partie hydrophile ou polaire et d'une partie lipophile ou apolaire. Ce sont des molécules amphiphiles puisqu'elles ont une double polarité. Les émulsionnants sont caractérisés par leur HLB ; si le HLB est élevé, la partie hydrophile est prédominante, si le HLB est faible, la partie lipophile prédomine.

Parmi ces émulsionnants, on inclut de façon préférée les émulsionnants polymériques qui se caractérisent par une masse molaire élevée et une structure non linéaire qui permet un ancrage plus important à l'interface eau/huile que celui obtenu avec les émulsionnants de type monomère.

Les émulsionnants qu'il est possible d'utiliser selon l'invention, seuls ou en mélange, sont ceux permettant de faire des émulsions inverses et ayant une HLB inférieure à 7.

D'une façon générale les émulsionnants préférés sont les émulsionnants siliconés, de type organopolysiloxanes tels que :
E1) Poly Alkyl méthicone Copolyols (Poly alkyl méthylsiloxane oxyalkylénés éventuellement réticulés) contenant :
   des chaînes Alkyle de C6 à C20, saturées ou non, linéaires ou ramifiées un motif Polyoxyéthyléné de 1 à 50 OE (Oxyde d'Ethylene)
      et/ou
   un motif Polyoxypropyléné de 1 à 50 OP (Oxyde de Propylène)
- E2) Poly Alkyl diméthyl méthylsiloxane oxyalkylénés contenant :
   des chaînes alkyle de C6 à C20, saturées ou non, linéaires ou ramifiées un motif Polyoxyéthyléné de 1 à 50 OE
      et/ou
   un motif Polyoxypropyléné de 1 à 50 OP

   Les organopolysiloxanes de la composition de l'invention contiennent notamment un ou plusieurs groupements oxyalkylénés et en particulier oxyéthylénés (OE), par exemple de 1 à 40 motifs oxyalkylénés, de préférence de 1 à 20, mieux de 10 à 20, de façon plus préférée de 12 à 20 et encore mieux de 12 à 18 motifs oxyalkylénés, pouvant former des chaînes polyoxyalkylènes et notamment polyoxyéthylènes. Ces groupements peuvent être pendants ou en bout de chaîné. Les atomes de silicium portant ces groupements sont avantageusement au nombre d'environ 1 à 10 et mieux de 1 à 6. La structure siliconée formant le squelette polymérique de l'organopolysiloxane à groupement(s) oxyalkyléné(s) est avantageusement une structure polydiméthylsiloxane (PDMS) dont éventuellement une partie des groupes méthyle est substituée par des groupements alkyle en C2 à C30 et de préférence en C8 à C24, et mieux de C10 à C20 ou phényle, soit en bout de chaîne soit pendants.
   Avantageusement, on utilisera donc comme émulsionnants de type E1 ou E2 les émulsionnants siliconés comme les alkyldiméthicone copolyols tels que l'Abil EM-90, ou le mélange de diméthicone copolyol et cyclométhicone, vendu par la société Dow Corning sous la dénomination 3225C Formulation Aid, le laurylmethicone copolyol vendu sous le nom d'Emulsifier 10 par Dow Corning, ou des mélanges à base d'un polymère siliconé tel que le cetyl dimethicone copolyol avec du polyglyceryl-4 isostéarate et de l'hexyl laurate vendu sous le nom d'Abil WE09 par la société Goldschmidt, l'Abil EM 97 dé Goldschmidt (Dimethicone copolyol & cyclomethicone), le Wacker SPG 128 VP de Wacker (cyclomethicone et octyldimethicone methoxy glycosyl), ou encore le Silwax WD-IS (Dimethicone copolyol iso-stearate)
E3) les mono ou polyalkylesters siloxanes, par exemple le Silwax S de Lamberit (Dimethiconol stearate),
E4) les esters d'acide alkoxylés carboxyliques comme les alkylesters polyhydroxylés de PEG, par exemple l'Arlacel P 135 de Uniqema (PEG-30 dipolyhydroxystearate).

On utilisera de préférence les émulsionnants de HLB compris entre 2 et 7, préférentiellement un émulsionnant E/H siliconé de HLB compris entre 2 et 7, préférentiellement un émulsionnant E/H siliconé polymérique de HLB compris entre 2 et 7.

La composition selon l'invention contiendra notamment, exprimé en pourcentage en poids, de 0,5 à 8% d'émulsionnant, par exemple de 0,5 à 5%, de préférence entre 3 et 5%, par rapport au poids total de la composition.

La composition selon l'invention comprend de préférence un émulsionnant siliconé choisi parmi le laurylméthicone copolyol, le cétyl diméthicone copolyol, un mélange de diméthicone copolyol et cyclométhicone ou un mélange de cétyl diméthicone copolyol avec du polyglycéryl-4 isostéarate et de l'hexyl laurate.

Par ailleurs, de façon avantageuse, pour améliorer la stabilité de la dispersion, il est possible d'associer aux émulsionnants principaux décrits plus haut un ou plusieurs co-émulsionnants ayant une HLB supérieure à 6. Le rapport (co-émulsionnant/émulsionnant) sera avantageusement inférieur à 1,5 et de préférence inférieur à 0,75.

A titre d'exemple, on peut citer :
- Les alkyl ou poly alkyl esters de sorbitan polyoxyéthylénés ou non avec entre 1 et 5 chaînes alkyles entre C10 et C20 saturées ou non, ramifiées ou non, et avec de 0 à 40 OE (par exemple : monolaurate de sorbitane 20 OE ou monooléate de sorbitan 20 OE (Tween 80 de Uniqema)) ;
- Les alkyl ou poly alkyl éthers ou esters polyoxyéthylénés avec entre 1 et 5 chaînes alkyles entre C10 et C20 saturées ou non, ramifiées ou non et avec de 0 à 40 OE (cétéareth-20 (Eumulgin B2 de Cognis), ou le stéareth (Brij 78) 20 OE) ;
- Les alkyl ou poly alkyl mono ou polyglucosides éthoxylés et estérifiés avec entre 1 et 5 chaînes alkyles entre C6 et C20 saturées ou non, ramifiées ou non et de 1 à 10 motifs de glucose (par exemple, le PEG-20 methylglucose sesqui stéarate (Glucamate SSE-20 de Amerchol)) ;
- Les Alkyl ou poly alkyl esters ou éthers de polyglycérol avec entre 1 et 5 chaînes alkyles entre C10 et C20 saturées ou non, ramifiées ou non et de 1 à 8 motifs glycérol (par exemple le polyglycéryl 4 - isostéarate ou le PEG-8 stéarate (Myrj 45)).

De préférence, la composition selon l'invention comprend également un co-émulsionnant ayant une HLB supérieure à 6, qui est de préférence le cétéareth-20.

Enfin, il est possible d'ajouter de façon avantageuse dans la phase dispersée, de 0,001 à 10% en poids par rapport au poids total de la formulation, d'un co-solvant de l'actif ayant une température d'évaporation inférieure à 100°C, de préférence des alcools linéaires ou ramifiés de C1 à C4, comme l'éthanol et l'isopropanol.

De façon intéressante, la préparation de l'émulsion selon l'invention s'est avérée ne nécessiter que peu d'énergie mécanique ou thermique par rapport aux préparations d'autres émulsions inverses déjà connues.

De façon connue, la composition de l'invention peut contenir également les adjuvants habituels dans les domaines cosmétique et dermatologique, tels que les gélifiants hydrophiles ou lipophiles, les humectants comme la glycérine et le sorbitol, les épaississants de phase grasse, les conservateurs les antioxydants, les électrolytes, les solvants, les parfums, les charges, les filtres, les pigments, les absorbeurs d'odeur, les matières colorantes et les agents chélatants des métaux. Les quantités de ces différents adjuvants sont celles classiquement utilisées dans les domaines considérés, et par exemple de 0,01 à 20 % du poids total de la composition. Ces adjuvants, selon leur nature, peuvent être introduits dans la phase lipophile ou dans la phase hydrophile Ces adjuvants, ainsi que leurs concentrations, doivent être tels qu'ils ne nuisent pas aux propriétés cosmétiques et/ ou dermatologiques de la composition selon l'invention.

Comme gélifiants hydrophiles, on peut citer en particulier les polymères carboxyvinyliques (carbomer), les copolymères acryliques tels que les copolymères d'acrylates/alkylacrylates ou les acrylates copolymères, les polyacrylamides, les polysaccharides, les gommes naturelles et les argiles, et, comme gélifiants lipophiles, on peut citer les argiles modifiées comme les bentones, les sels métalliques d'acides gras, la silice hydrophobe, les gommes de silicone. De préférence selon l'invention, on utilisera les gélifiants commerciaux suivants :
- le Lubrajel vendu par la société Guardian, qui est un mélange de Glyceryl polymethacrylate et de propylène glycol,
- l'Aculyn 44 vendu par la société Rohm et Haas, qui est un mélange de PEG-150, d'alcool décylique et de copolymère SMDI

La composition selon l'invention a un toucher cosmétiquement acceptable, une bonne tolérance cutanée, une bonne stabilité physique, c'est à dire absence de déphasage et maintien de la taille des globules au froid (à 4°C) et à la chaleur (45°C) sur une longue durée, par exemple sur 3 mois, avec une viscosité stable sur cette période. La composition selon l'invention permet également de conférer à l'actif une bonne stabilité chimique et d'éviter sa cristallisation dans le temps.

En particulier, l'invention concerne une composition cosmétique ou dermatologique pour application topique sur la peau, les phanères et/ou les muqueuses, sous forme d'une émulsion inverse contenant une phase hydrophile glycolique ou hydroglycolique dispersée et une phase continue lipophile, caractérisée en ce qu'elle comprend, dans un milieu physiologiquement acceptable (c'est-à-dire compatible avec l'application topique sur la peau, les phanères et/ou les muqueuses), exprimé en pourcentage en poids par rapport au poids total de la composition :
- de 0,01 à 5% d'ivermectine,
- de 30 à 70% de glycols,
- de 0,5 à 8% d'émulsionnant de HLB compris entre 2 et 7,
- de 0% à 5% de coémulsionnant de HLB supérieur à 6,
- de 0 à 50% d'eau, de préférence de 0 à 25% d'eau.

Dans un mode de réalisation particulier de l'invention, la phase hydrophile dispersée a une activité en eau inférieure à 0,85.

Les compositions selon l'invention sont notamment utiles à titre de médicament.

L'invention a également pour objet l'utilisation de la composition selon l'invention pour la fabrication d'un médicament destiné à prévenir et/ou traiter les affections dermatologiques choisies parmi la rosacée, l'acné vulgaire, la dermite seborrheique, la dermatite periorale, les éruptions acneiformes, la dermatite acantholytique transitoire et l'acné miliaris necrotica.

La composition sous forme d'émulsion inverse et contenant de préférence de l'ivermectine selon l'invention, est plus particulièrement destinée au traitement de la rosacée.

L'invention couvre également les préparations pharmaceutiques et les médicaments obtenus à partir des compositions selon l'invention.

L'invention sera maintenant illustrée par les exemples non limitatifs suivants.

### EXEMPLE 1: méthodes de préparation des compositions des exemples 2 et 3 selon l'invention

Dans les compositions ci-après (Exemples 2 à 3) les proportions des différents constituants sont exprimées en pourcentages en poids, sauf indication contraire.

Les compositions des exemples 2 et 3 sont préparées de la façon suivante

### Phase grasse A

Peser les constituants de la phase grasse A et chauffer à 55°C.

### Phase B

Solubiliser l'Ivermectine dans le propylène Glycol chauffé à 55°C. Incorporer les autres constituants

### Pré-Emulsification

Introduire lentement la phase B dans la phase grasse A, sous agitation modérée.

### Refroidissement

Laisser sous agitation jusqu'à température ambiante.

### Phase C

Solubiliser l'électrolyte dans l'eau.

### Phase D (si applicable)

Ajout de la phase D à la phase C.

### Emulsification

Introduire lentement la phase aqueuse (C+D) dans la pré-émulsion, sous agitation modérée.

### Exemple 2 : Composition

| Phase A | |
|---|---|
| Laurylmethicone copolyol | 3,00% |
| Cyclopentasiloxane | 6,00% |
| Mineral oil | 15,00% |
| Butylhydroxytoluène | 0,10% |

| Phase B | |
|---|---|
| Propylene Glycol | Qsp 100% |
| Glyceryl Polymethacrylate (and) Propylene Glycol | 5,00% |
| Ivermectine | 1.00% |

| Phase C | |
|---|---|
| Eau Purifiée | 20,00% |
| Magnésium Sulfate, 7H2O | 1,00% |

### Exemple 3 : Composition

| Phase A | |
|---|---|
| Laurylmethicone copolyol | 3,00% |
| Cyclopentasiloxane | 6,00% |
| Mineral oil | 10,00% |
| Butylhydroxytoluène | 0,10% |

| Phase B | |
|---|---|
| Propylene Glycol | Qsp 100% |
| Glyceryl Polymethacrylate (and) Propylene Glycol | 5,00% . |
| Ivermectine | 1.00% |

| Phase C | |
|---|---|
| Eau Purifiée | 14,00% |
| Magnésium Sulfate, 7H2O | 1,00% |

| Phase D | |
|---|---|
| Ethanol Rectapur | 5,00% |

### Exemple 4 Résultats de stabilité de la composition selon l'exemple 2

### a) Stabilité physique

La stabilité physique des formulations est mesurée par une observation macroscopique et microscopique de la formulation à température ambiante, à 4°C , à 40°C et à 55°C après 1, 2, 3, voire 6 et 9 mois.

A température ambiante (TA), l'observation macroscopique permet de garantir l'intégrité physique des produits et l'observation microscopique permet de vérifier qu'il n'y a pas recristallisation de l'actif solubilisé.

A 4°C, l'observation microscopique vérifie la non-recristallisation des actifs solubilisés. A 40°C et/ou à 55°C, l'observation macroscopique vérifie l'intégrité du produit fini.

### Spécifications à T0, à TA de la composition selon l'exemple 2

*Aspect macroscopique :* Lait épais incolore ou très légèrement jaune.
*Aspect microscopique :* taille des globules d'ivermectine entre 2.5µ et 12.5µ.

| Temps→ | T 1M | T 2M | T3M |
|---|---|---|---|
| Conditions de stabilité | | | |
| ↓ | | | |
| TA | Conforme aux spécifications | Conforme aux spécifications | Conforme aux spécifications |
| +4°C | Conforme aux spécifications | Globules de 2.5µ à 25µ | Globules 2.5µ à 25µ |
| +55°C | Conforme aux spécifications | Globules de 2.5µ à 25µ | Globules de 2.5µ à 25µ |

Mesures de la viscosité (τ= contrainte de cisaillement) :
- Tinitial (T0) :: A 4 s-1, τ= 34 Pa
A 20 s-1, τ= 87 Pa
- T3mois/TA:: A 4 s-1, τ= 33 Pa
A 20 s-1, τ= 75 Pa
- T3 mois /40°C:: A 4 s-1, τ= 36 Pa
A 20 s-1, τ=81 Pa

Ces résultats montrent une très bonne reproductibilité et une excellente stabilité sur 3 mois du profil rhéologique de la formulation selon l'invention.

### b) Stabilité chimique de l'actif au sein de la composition selon l'exemple 2

Le dosage de l'actif est effectué par étalonnage interne en HPLC.

| | **TA** | **40°C** |
|---|---|---|
| T0 | 97.7 % | |
| T1mois | 97.8% | 97.9% |
| T2mois | 99.2% | 98.5% |
| T3mois | 99.2% | 98.4% |
| T6mois | 99.1% | 98.6% |

Ces résultats montrent que l'actif au sein de la composition et la composition elle-même présentent une grande stabilité.

### Exemple 5 : Résultats de stabilité de la composition selon l'exemple 3

### a) Stabilité physique

### Spécifications à T0, à TA de la composition selon l'exemple 3

*Aspect macroscopique :* crème souple incolore ou très légèrement jaune.
*Aspect microscopique :* taille des globules d'ivermectine entre 2.5µ et 12.5µ.

| Temps→ | T1M | T2M | T3M |
|---|---|---|---|
| Conditions de stabilité | | | |
| ↓ | | | |
| TA | Conforme aux spécifications | Conforme aux spécifications | Conforme aux spécifications |
| +4°C | Conforme aux spécifications | Globules de 2.5µ à 25µ | Globules 2.5µ à 25µ |
| +55°C | Conforme aux spécifications | Globules de 2.5µ à 25µ | Globules de 2.5µ à 25µ |

### b) Stabilité chimique de l'actif au sein de la composition selon l'exemple 3

Le dosage de l'actif est effectué par étalonnage interne en HPLC.

| | **TA** | **40°C** |
|---|---|---|
| T0 | 100.7% | / |
| T1 mois | 100.7% | 101.1% |
| T2mois | 101.4% | 102.6% |
| T3mois | 101.0% | 107.9% |
| T6mois | 101.0% | 98.6% |
| T9mois | 98.4% | / |
| T12mois | 97.9% | / |

Ces résultats montrent que l'actif au sein de la composition et la composition elle-même présentent une grande stabilité.

### Exemple 6 : Evaluation de la tolérance de la composition selon l'exemple 2 par un test de tolérance locale après application répétée chez la souris.

La présente étude a pour but de comparer le pouvoir irritant de la composition placébo selon l'invention avec différentes compositions placébo d'ivermectine.

Le traitement consiste en une application topique quotidienne d'une composition sur la face interne de l'oreille droite de souris BALB/c pendant 6 jours.

Les produits à tester sont :
Groupe 1 : Formule crème A
Groupe 2 : Formule crème B
Groupe 3 : Formule Gel-crème C
Groupe 4 : Formule Gel D
Groupe 5 : Emulsion E
Groupe 6 : Emulsion inverse selon l'invention F

L'évaluation se fait par des mesures de l'épaisseur de l'oreille à l'aide de l'Oditest et par observation clinique des animaux du 2ème au 12ème jour.
Les résultats sont :

| | AUC d'oedème D2-D19 | | % inhibition AUC | P valeurs | t-test Student |
|---|---|---|---|---|---|
| | | | | | vs non traité |
| | Moyenne | sem | vs Differin | | |
| Non traité | 209.4 | 1.8 | | | |
| Groupe 1 : | | | | | |
| Formule crème A | 238.5 | 2.8 | **13.9** | **0.0000** | ******* |
| Groupe 2 : | | | | | |
| Formule crème B | 243.5 | 4.4 | **16.3** | **0.0001** | ******* |
| Groupe 3 : | | | | | |
| Formule Gel-crème C | 227 | 5.8 | **8.4** | **0.206** | ***** |
| Groupe 4 : | | | | | |
| Formule Gel D | 306.1 | 6.7 | **46.2** | **0.0000** | ******* |
| Groupe 5 : | | | | | |
| Emulsion E | 228.6 | 2.5 | **9.2** | **0.0002** | ******* |
| Groupe 6 : | | | | | |
| Emulsion inverse selon l'invention F | 208.8 | 2.4 | **-0.3** | **0.8473** | **NS** |

La formulation selon l'invention n'a pas induit d'augmentation de l'épaisseur de l'oreille. Cette formulation est donc considérée comme très bien tolérée chez la souris et de façon significativement différente des autres compositions testées.

## Revendications

1. Composition comprenant au moins un composé de la famille des avermectines, **caractérisée en ce que** la composition est une émulsion inverse contenant une phase hydrophile dispersée glycolique ou hydroglycolique, une phase continue lipophile et un émulsionnant de HLB compris entre 2 et 7, ladite composition ne comprenant pas de DHEA et/ou ses précurseurs et/ou dérivés chimiques et/ou biologiques et/ou de dérivé de vitamine D.

2. Composition selon la revendication 1, **caractérisée en ce que** le composé de la famille des avermectines est l'ivermectine.

3. Composition selon la revendication 1 ou 2, **caractérisée en ce que** le composé de la famille des avermectines est solubilisé dans la phase glycolique ou hydroglycolique dispersée.

4. Composition selon l'une des revendications 1 à 3, **caractérisée en ce que** le composé de la famille des avermectines est présent en quantité comprise entre 0.01 et 5 % en poids par rapport au poids total de la composition, préférentiellement entre 0,02 et 2%.

5. Composition selon l'une des revendications 2 à 4 **caractérisée en ce qu'**elle comprend 1% d'ivermectine.

6. Composition selon l'une des revendication 1 à 5, **caractérisée en ce que** l'émulsionnant est un émulsionnant siliconé.

7. Composition selon l'une des revendication 1 à 6, **caractérisée en ce que** l'émulsionnant est choisi parmi le laurylméthicone copolyol, le cétyl diméthicone copolyol, un mélange de diméthicone copolyol et cyclométhicone ou un mélange de cétyl diméthicone copolyol avec du polyglycéryl-4 isostéarate et de l'hexyl laurate.

8. Composition selon l'une des revendications 1 à 7, **caractérisée en ce qu'**elle comprend également un co-émulsionnant ayant une HLB supérieure à 6.

9. Composition selon la revendication 8, **caractérisée en ce que** le co-émulsionnant est le cétéareth-20..

10. Composition selon l'une des revendications 1 à 9, **caractérisée en ce que** la proportion volumique de glycol par rapport au volume total de la phase dispersée, est comprise entre 60 et 100%.

11. Composition selon l'une des revendications 1 à 10, **caractérisée en ce que** la phase dispersée comprend au moins un glycol choisi parmi le propylène glycol, l'hexylène glycol, le dipropylène glycol et le PEG 400.

12. Composition selon l'une des revendications 1 à 11, **caractérisée en ce qu'**elle comprend, en pourcentage en poids par rapport au poids total de la composition :
- de 0,01 à 5%, d'ivermectine,
- de 30 à 70% de glycols,
- de 0,5 à 8% d'émulsionnant de HLB compris entre 2 et 7,
- de 0% à 5% de co-émulsionnant de HLB supérieur à 6,
- de 0 à 50% d'eau.

13. Composition selon l'une des revendications 1 à 12 à titre de médicament.

14. Utilisation d'une composition selon l'une quelconque des revendications 1 à 12 pour la préparation d'un médicament destiné à la prévention et/ou au traitement d'un désordre choisi parmi la rosacée, l'acné vulgaire, la dermite seborrheique, la dermatite periorale, les éruptions acneiformes, la dermatite acantholytique transitoire, et l'acné miliaris necrotica.

15. Utilisation selon la revendication 14, **caractérisée en ce que** le désordre est la rosacée.

## Claims

1. Composition comprising at least one compound of the family of the avermectins, **characterized in that** the composition is an inverse emulsion comprising a glycolic or aqueous/glycolic dispersed hydrophilic phase, a continuous lipophilic phase and an emulsifier with an HLB of between 2 and 7, said composition not comprising DHEA and/or its precursors and/or chemical and/or biological derivatives and/or vitamin D derivative.

2. Composition according to Claim 1, **characterized in that** the compound of the family of the avermectins is ivermectin.

3. Composition according to Claim 1 or 2, **characterized in that** the compound of the family of the avermectins is dissolved in the dispersed glycolic or aqueous/glycolic phase.

4. Composition according to one of Claims 1 to 3, **characterized in that** the compound of the family of the avermectins is present in an amount of between 0.01 and 5% by weight, with respect to the total weight of the composition, preferably between 0.02 and 2% by weight.

5. Composition according to one of Claims 2 to 4, **characterized in that** it comprises 1% of ivermectin.

6. Composition according to one of Claims 1 to 5, **characterized in that** the emulsifier is a silicone emulsifier.

7. Composition according to one of Claims 1 to 6, **characterized in that** the emulsifier is chosen from laurylmethicone copolyol, cetyldimethicone copolyol, a mixture of dimethicone copolyol and cyclomethicone or a mixture of cetyldimethicone copolyol with polyglyceryl-4 isostearate and hexyl laurate.

8. Composition according to one of Claims 1 to 7, **characterized in that** it also comprises a coemulsifier having an HLB of greater than 6.

9. Composition according to Claim 8, **characterized in that** the coemulsifier is ceteareth-20.

10. Composition according to one of Claims 1 to 9, **characterized in that** the proportion by volume of glycol, with respect to the total volume of the dispersed phase, is between 60 and 100%.

11. Composition according to one of Claims 1 to 10, **characterized in that** the dispersed phase comprises at least one glycol chosen from propylene glycol, hexylene glycol, dipropylene glycol and PEG 400.

12. Composition according to one of Claims 1 to 11, **characterized in that** it comprises, as percentage by weight with respect to the total weight of the composition:
- from 0.01 to 5% of ivermectin,
- from 30 to 70% of glycols,
- from 0.5 to 8% of emulsifier with an HLB of between 2 and 7,
- from 0 to 5% of coemulsifier with an HLB of greater than 6,
- from 0 to 50% of water.

13. Composition according to one of Claims 1 to 12 as medicament.

14. Use of a composition according to any one of Claims 1 to 12 in the preparation of a medicament intended for the prevention and/or treatment of a disorder chosen from rosacea, acne vulgaris, seborrheic dermatitis, perioral dermatitis, acneiform eruptions, transient acantholytic dermatosis and acne miliaris necrotica.

15. Use according to Claim 14, **characterized in that** the disorder is rosacea.

## Patentansprüche

1. Zusammensetzung, die mindestens eine Verbindung der Avermectin-Familie umfasst, **dadurch gekennzeichnet, dass** es sich bei der Zusammensetzung um eine Umkehremulsion handelt, die eine dispergierte hydrophile Glykolphase oder wässrige Glykolphase, eine geschlossene lipophile Phase und einen Emulgator mit einem HLB-Wert zwischen 2 und 7 enthält, wobei die Zusammensetzung kein DHEA und/oder seine Vorstufen und/oder chemischen Derivate und/oder biologischen Derivate und/oder Vitamin-D-Derivate umfasst.

2. Zusammensetzung nach Anspruch 1, **dadurch gekennzeichnet, dass** es sich bei der Verbindung aus der Avermectin-Familie um Ivermectin handelt.

3. Zusammensetzung nach Anspruch 1 oder 3, **dadurch gekennzeichnet, dass** die Verbindung der Avermectin-Familie in der dispergierten Glykolphase oder wässrigen Glykolphase solubilisiert ist.

4. Zusammensetzung nach einem der Ansprüche 1 bis 3, **dadurch gekennzeichnet, dass** die Verbindung der Avermectin-Familie in einer Menge zwischen 0,01 und 5 Gew.-% in Bezug auf das Gesamtgewicht der Zusammensetzung, vorzugsweise zwischen 0,02 und 2%, vorliegt.

5. Zusammensetzung nach einem der Ansprüche 2 bis 4, **dadurch gekennzeichnet, dass** sie 1% Ivermectin umfasst.

6. Zusammensetzung nach einem der Ansprüche 1 bis 5, **dadurch gekennzeichnet, dass** es sich bei dem Emulgator um einen Silikonemulgator handelt.

7. Zusammensetzung nach einem der Ansprüche 1 bis 6, **dadurch gekennzeichnet, dass** der Emulgator aus der Gruppe Laurylmethicon-Copolyol, Cetyldimethicon-Copolyol, einem Gemisch von Dimethicon-Copolyol und Cyclomethicon oder einem Gemisch von Cetyldimethicon-Copolyol mit Polyglyceryl-4-Isostearat und Hexyllaurat ausgewählt ist.

8. Zusammensetzung nach einem der Ansprüche 1 bis 7, **dadurch gekennzeichnet, dass** sie weiterhin einen Coemulgator mit einem HLB-Wert von über 6 umfasst.

9. Zusammensetzung nach Anspruch 8, **dadurch gekennzeichnet, dass** es sich bei dem Coemulgator um Ceteareth-20 handelt.

10. Zusammensetzung nach einem der Ansprüche 1 bis 9, **dadurch gekennzeichnet, dass** der volumenbezogene Mengenanteil an Glykol bezogen auf das Gesamtvolumen der dispersen Phase zwischen 60 und 100% beträgt.

11. Zusammensetzung nach einem der Ansprüche 1 bis 10, **dadurch gekennzeichnet, dass** die disperse Phase mindestens ein Glykol umfasst, das aus der Reihe Propylenglykol, Hexylenglykol, Dipropylenglykol und PEG 400 ausgewählt ist.

12. Zusammensetzung nach einem der Ansprüche 1 bis 11, **dadurch gekennzeichnet, dass** sie als Gewichtsprozent in Bezug auf das Gesamtgewicht der Zusammensetzung
- 0,01 bis 5% Ivermectin,
- 30 bis 70% Glykole,
- 0,5 bis 8% Emulgator mit einem HLB-Wert zwischen 2 und 7,
- 0% bis 5% Coemulgator mit einem HLB-Wert über 6,
- 0 bis 50% Wasser
umfasst.

13. Zusammensetzung nach einem der Ansprüche 1 bis 12 als Arzneimittel.

14. Verwendung einer Zusammensetzung nach einem der Ansprüche 1 bis 12 zur Herstellung eines Arzneimittels zur Vorbeugung und/oder Behandlung einer Krankheit, ausgewählt aus der Reihe Rosacea, Akne vulgaris, Dermatitis seborrhoica, periorale Dermatitis, akneiformen Eruptionen, transitorische akantholytische Dermatose sowie Acne miliaris necrotica.

15. Verwendung nach Anspruch 14, **dadurch gekennzeichnet, dass** es sich bei der Krankheit um Rosacea handelt.
